# EUROPÄISCHE PATENTSCHRIFT

(11) **EP 2 257 338 B1**
(45) Veröffentlichungstag und Bekanntmachung des Hinweises auf die Patenterteilung: **27.02.2013**
(21) Anmeldenummer: 08873599.8
(22) Anmeldetag: 28.11.2008
(51) Int. Cl.: A61N 5/06, A61M 21/00

(54) **VORRICHTUNG ZUR STABILISIERUNG UND MODIFIZIERUNG BIOLOGISCHER RHYTHMEN UND ZUR BEHANDLUNG VON RHYTHMUSSTÖRUNGEN**
DEVICE FOR STABILIZING AND MODIFYING BIOLOGICAL RHYTHMS AND FOR TREATING RHYTHM DISTURBANCES
DISPOSITIF POUR STABILISER ET MODIFIER LES RYTHMES BIOLOGIQUES ET POUR TRAITER LES ARYTHMIES

(30) Priorität: 22.03.2008 WO PCT/DE2008/000495
(43) Veröffentlichungstag der Anmeldung: 08.12.2010
(73) Patentinhaber: Turbolite Vertriebs Gmbh, 12623 Berlin (DE)
(72) Erfinder: STEINBRÜCK, Volker, 13125 Berlin (DE)
(74) Vertreter: Ninnemann, Detlef
(86) Internationale Anmeldenummer: PCT/EP2008/066401
(87) Internationale Veröffentlichungsnummer: WO 2009/118066

(56) Entgegenhaltungen:
- WO-A-2007/091188
- US-A- 5 634 711
- US-A- 5 923 398
- US-A1- 2007 185 553
- US-B1- 6 350 275

## Beschreibung

Die Erfindung betrifft eine optoelektrische Vorrichtung zur Einstrahlung von Licht in mindestens ein Auge eines Anwenders gemäß dem Oberbegriff des Anspruchs 1.

Es ist allgemein bekannt, dass in den letzten Jahren die Kenntnisse der Photobiologie des Menschen, insbesondere die Kenntnisse der Wirkung von Lichtstrahlung auf menschliche Probanden, stark angewachsen sind. Alle Aussagen stützten sich vormals großteils auf die Untersuchung der Einwirkung "weißen" Lichtes variierender Intensität auf das menschliche Auge. Die Verwendung von konventionellen "weißen" Lichtquellen wie Temperaturstrahlem und Leuchtstofflampen in der Phototherapie ist schon länger bekannt. Unterschiedliche Geräte nutzten die phototherapeutische Wirkung des "weißen" Lichtes bei Applikation ins menschliche Auge, siehe hier beispielsweise US 5,503,637; US 6,053,936; WO 91 14 475; WO 90 10 473; WO 89 08 476; DE 295 14 342 U1.

Die phototherapeutische Einflussnahme zielt im Wesentlichen auf die zirkadiane Rhythmik im Leben der Probanden, entweder zur Stabilisierung der Rhythmik oder zu deren gezielten Veränderung. Die zirkadiane Rhythmik ist elementar wichtig für den menschlichen Körper und steuert im tag-/nächtlichen Takt den Blutdruck, die Körpertemperatur, den Wach-Schlaf Rhythmus und viele andere Körperfunktionen des Menschen.

Bekannt und gut untersucht ist die Synchronisation der zirkadianen Rhythmik durch das Tageslicht, desgleichen die Möglichkeit der Modifikation durch weißes Kunstlicht. Die Applikation weißen Lichts zur Beeinflussung der zirkadianen Rhythmik ist Stand der Technik, obwohl seit längerem bekannt ist, dass ein blaues Teilspektrum des weißen Lichtes gleiche Wirkungen auf den Wach-/Schlaf Zyklus und auf die Müdigkeit ausübt. (Siehe dazu z. B. Annals New York Academia of Sciences 453 (1985) S. 376-378 und Journal of Neuroscience, No. 21 (16), 2001 S. 6.405-6.412, sowie Journal of Physiology, No. 535.1, 2001, S. 261-267.) Als Indikator für die Licht- Wirkung wurde der Melatoningehalt im Blutplasma gewählt. Melatonin ist ein Hormon, das im menschlichen Körper einen spezifischen Tageszyklus aufweist und als einer der Marker des menschlichen Bio-Rhythmus in der Forschung anerkannt ist. Es wurde herausgefunden, dass bei hinreichender Intensität ein bestimmter sogenannter zirkadian-wirksamer Wellenlängenbereich des sichtbaren Lichtes (nämlich der Bereich von ca. 400 bis 520 nm) bei Anwendung auf das Auge menschlicher Probanden zu einer besonders ausgeprägten Melatoninunterdrückung führt, wobei gezeigt wurde, dass ein vergleichsweise niedriger Melatoninspiegel Ausdruck gesteigerter Wachheit ist und ein vergleichsweise hoher Spiegel Ausdruck gesteigerter Schläfrigkeit ist. Man spricht in diesem Zusammenhang von der zirkadianen Empfindlichkeitskurve des Menschen, die als Funktion der Wellenlänge angibt, wie stark die durch die Lichtstrahlung induzierte Wirksamkeit des Lichtes auf den menschlichen Bio-Rhythmus ist. Die DE 601 20 430 T2 beschreibt ein Verfahren zur Steuerung der Wachheit eines Menschen mittels blauer Lichtstrahlung.

Die Änderung des Melatoninspiegel eines Menschen ist allerdings nur einer unter mehreren Indikatoren für die Wirkung auf den zirkadianen Rhythmus durch Bestrahlung eines Probanden mit Licht. Darüber hinaus hat der Erfinder und andere in Versuchsreihen ermittelt, dass eine spezifische Bestrahlung mit Licht im Wellenlängenlängenbereich von 400 bis 520 nm (sog. "blaues" Licht) nicht nur den Wach/Schlaf Rhythmus beeinflusst und eine Verringerung der Müdigkeit herbeiführt, sondern auch die allgemeine Befindlichkeit (Stimmungslage, Ruhe/Unruhe-Status) beeinflusst. Durch Applikation dieser Strahlung können nicht nur Veränderungen des 24-Stunden-Tag-Nacht-Rhythmus des Menschen hervorgerufen werden (dies schließt insbesondere auch die Behebung von Störungen dieses Rhythmus ein), es können auch länger- oder kürzerperiodische Rhythmen sowie Stimmungsschwankungen und der Grad der psychologischen Anspannung beeinflusst werden.

Es konnten experimentelle Bedingungen gezeigt werden, unter denen die positiven Wirkungen blauen Lichts diejenigen weißen Lichts übertreffen. "Blaues" Licht wurde von den Probanden nicht nur subjektiv als angenehmer empfunden, sondern führt zu messbaren Entspannungssteigerungen.

Aus der US-B1-6 350 275 ist als nächstliegender Stand der Technik eine gattungsgemäße Vorrichtung bekannt, bei der ein herkömmliches Brillengestell so modifiziert ist, dass eine oder mehrere kleine LEDs so auf der Brille angebracht sind, dass die LEDs Licht ins Auge strahlen können, wenn die Vorrichtung als Brille getragen wird.

Nachteilig an der bekannten Vorrichtung ist unter anderem, dass die Anbringung von LEDs an einem Brillengestell erfolgt, so dass sich das Gerät nicht bei Menschen bestimmungsgemäß zur Anwendung bringen lässt, während diese eine andere Vorrichtung, wie zum Beispiel eine andere Brille zur Korrektur von Fehlsichtigkeit, verwenden. Außerdem schränkt die Vorrichtung einen erheblichen Teil des Sichtfeldes des Anwenders ein.

Der vorliegenden Erfindung liegt die Aufgabe zugrunde, eine optoelektrische Vorrichtung zur Einstrahlung von Licht in mindestens ein Auge eines Anwenders bereit zu stellen, die kompakt und ergometrisch gestaltet und universell einsetzbar ist.

Erfindungsgemäß wird diese Aufgabe durch eine Vorrichtung mit den Merkmalen des Anspruchs 1 gelöst.

Die erfindungsgemäße Lösung gewährleistet durch die Anordnung der Bestandteile der Vorrichtung dergestalt, dass das Sichtfeld des Anwenders bei bestimmungsgemäßem Anwenden der Vorrichtung zusammen mit dem bestimmungsgemäßen Anwenden einer Brille genauso oder nur geringfügig mehr eingeschränkt ist als durch bestimmungsgemäßes Anwenden der Brille ohne die Vorrichtung, eine kompakte und ergometrische Gestaltung der optoelektrischen Vorrichtung zur Einstrahlung von Licht in mindestens ein Auge eines Anwenders.

Vorteilhafte und bevorzugte Ausgestaltungen der Erfindung sind in den Unteransprüchen angegeben.

In einer vorteilhaften Ausgestaltungsform ist mindestens eine der Strahlungsquellen eine LED.

Die Strahlungsquellen sind so gewählt, dass der höchste Anteil der Strahlungsintensität im zirkadian-wirksamen Spektrum liegt. Insbesondere Strahlungsquellen mit einem Maximum der Strahlungsintensität bei einer Wellenlänge oder einem Bereich von Wellenlängen nahe am Maximum der zirkadianen Empfindlichkeits-Kurve sind hier als besonders wirksam vorteilhaft. Diese Bauart der Vorrichtung erlaubt, dass ein großer Anteil der Energie, die die Stromversorgung zur Verfügung stellt, ausschließlich zur Erzeugung von Licht im zirkadian-wirksamen Spektrum verwendet wird. Der geringere Energieverbrauch für die Beeinflussung des Melatoninspiegels erlaubt eine Stromversorgung mit weniger Leistung als bei herkömmlichen Strahlungsquellen zur Bewirkung eines gleichwirkenden Effekts auf den Bio-Rhythmus. Dies wiederum hat den Vorteil, eine kompakte Bauweise möglich zu machen, da die Energiequelle und Stromversorgung räumlich kompakter angelegt werden können.

Die optoelektrische Vorrichtung kann an einem Gegenstand angeordnet oder befestigt werden. Diese Anordnung kann so erfolgen, dass die Vorrichtung an dem Gegenstand fest angeordnet ist oder so, dass die Anordnung nicht fest ist und eine Demontage ohne Hilfsmittel möglich ist. Eine weitere Ausführungsvariante ist die Anordnung dieses Gegenstandes am Kopf des Anwenders. Insbesondere eine Befestigung oder Anordnung an einem Stirnband oder einer Kopfbedeckung, die am beziehungsweise auf dem Kopf getragen werden können, sind Ausführungsbeispiele.

Die Vorrichtung umfasst mindestens eine optische Komponente, die sich dadurch auszeichnet, dass sie die Strahlung streut und/oder beugt und/oder lenkt und/oder reflektiert. Die optische Komponente streut und/oder beugt und/oder lenkt und/oder reflektiert insbesondere einen Anteil der Strahlung im zirkadian-wirksamen Emissions-Wellenlängen-Bereich der Strahlungsquelle. Hierdurch kann eine effiziente Nutzung der Energie, die für das Betreiben der mindestens einen Strahlungsquelle bereitzustellen ist, für die Beeinflussung von Bio-Rhythmen erreicht werden.

Das Gestell und die Anordnung der weiteren Bestandteile der Vorrichtung ist dergestalt, dass in oder an der Vorrichtung mindestens ein Abstandhalter ausgestaltet ist, so dass die Vorrichtung so am Kopf des Anwenders tragbar ist, dass ein Abstand oder Abstandsbereich zwischen Strahlungsquelle sowie dem Auge des Anwenders festgelegt ist und/oder ein Winkel oder Winkelbereich zwischen mindestens einer Strahlungsrichtung der mindesten einen Strahlungsquelle und dem Auge des Anwenders festgelegt ist. Insbesondere kann dies durch einen Abstandshalter im oder am Gestell der Vorrichtung erreicht werden. Ein solcher Abstandshalter umfasst in einer Ausführungsform ein Nasenbügel, der auf dem Nasenrücken des Anwenders postierbar ist.

Die Stromversorgung der optoelektrischen Vorrichtung umfasst eine wieder aufladbare Energiequelle mit einem Konstantstrom-Regler und/oder mit einer Zwangsabschaltungseinheit. In einer weiteren Ausführung ist mindestens ein Lichtaustrittsfenster für die Strahlung der Strahlungsquelle im oder am Gestell angeordnet. Weiterhin umfasst die Vorrichtung mindestens ein Bedienelement, das im oder am Gestell angeordnet ist. So kann erreicht werden, dass der Anwender die Vorrichtung durch Betätigen eines Bedienelements im oder am Gestell steuern kann. In einer Ausführungsvariante umfasst die Vorrichtung mindestens ein Signallicht. Dieses Signallicht ist vorteilhaft eine LED. Das Abstrahlungsspektrum des Signallichtes unterscheidet sich vorteilhaft von dem der zirkadian-wirksamen Strahlungsquelle. Damit kann die vom Signallicht ausgesandte Strahlung Informationen über den Funktionszustand des Gerätes übermitteln, ohne mit der zirkadian-wirksamen Strahlungsquelle verwechselt zu werden.

In einer Ausführungsform ist eine elektrische Verbindung zu einer äußeren Stromversorgung bereitgestellt. Die Verbindung erfolgt so, dass die Energiequelle der inneren Stromversorgung durch die Energie der äußeren Stromversorgung wieder aufladbar ist. Eine Ladevorrichtung für die Energiequelle der Stromversorgung ist im oder auf dem Gestell angeordnet. In vorteilhaften Ausführungsformen ist die Stromversorgung der erfindungsgemäßen Vorrichtung mindestens ein Akkumulator und/oder Lithium-Akkumulator und/oder Lithium-Polymer-Akkumulator. Eine Ausführungsform der erfindungsgemäßen Vorrichtung umfasst vorteilhaft eine Ladeüberwachung und/oder Entladeüberwachung und/oder Sicherheitsabschaltung.

In einer Ausführungsvariante umfasst die Vorrichtung eine Steuereinheit, die die Bestrahlungszeit und/oder die Strahlungsintensität steuert.

Durch die Steuereinheit ist sichergestellt, dass bei bestimmungsgemäßem Gebrauch der Vorrichtung keine Gefährdung des Anwenders durch die Strahlung auftreten kann.

Die Vorrichtung ist so ausgestaltet, dass mindestens eine Strahlungsquelle mit Gleichstrom oder mit einem pulsweitenmodulierten Strom (PWM-Strom) betreibar ist.

In einer vorteilhaften Ausgestaltungsform umfasst die Vorrichtung mindestens einen USB-Port. Dieser kann zum Beispiel zum Laden der Energiequelle der Stromversorgung oder zur Kommunikation der Vorrichtung mit externen Einrichtungen verwendet werden.

Nach vorliegenden neuen Erkenntnissen kann "blaues" Licht auch zur Behandlung des prämenstruellen Syndroms, zur Behandlung einiger Formen von Depressionen und von saisonal affektive Störungen eingesetzt werden.

Die Vorrichtung wird daher zur Behandlung des prämenstruellen Syndroms, von Depressionen, insbesondere auch saisonal affektiver Störungen, oder Kopfschmerzen, insbesondere Migräne, sowie biologischer Rhythmusstörungen bei mindestens einem Probanden verwendet. Natürlich ist die Verwendung der Vorrichtung nicht auf die oben genannten Beschwerdemerkmale beschränkt, sondern kann darüber hinaus zur Steigerung des allgemeinen Wohlbefindens oder zur Kontrolle der Wachheit des Probanden oder in anderer Weise eingesetzt werden.

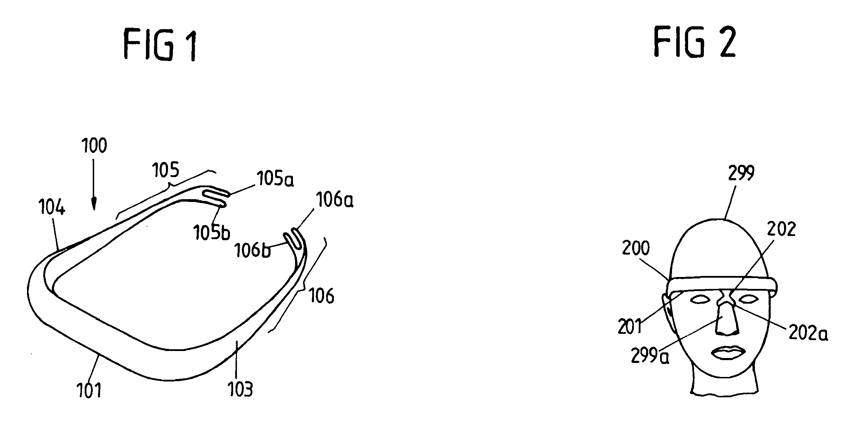

In Fig. 1 ist ein vorteilhaftes Ausführungsbeispiel des Gestells 100 einer erfindungsgemäßen Vorrichtung abgebildet. Das Gestell 100 in der Abbildung ist ein Gegenstand, der zur Anordnung der Vorrichtung am Kopf des Anwenders dient. Das Gestell 100 umfasst in der abgebildeten Ausgestaltungsform insbesondere einen Mittelteil 101, der etwa so lang wie die durchschnittliche Stimbreite eines Anwenders ist. Die Breite des Mittelteils 101 ist so bestimmt, dass in oder an ihm die Strahlungsquelle angeordnet werden kann. An jeweils einem Ende des Mittelteiles 101 schließen je ein Seitenbügelteil 103 und 104 an, diese sind ungefähr senkrecht zum Mittelteil 101 in die gleiche Richtung weisend angeordnet. Entlang der Richtung dieser Seitenbügelteile 103, 104 sind jeweils anschließend je ein Abschlussteil 105, 106 angeordnet. Dieses Abschlussteil 105, 106 weist in einem direkt an den Seitenbügelteil 103, 104 angrenzenten Bereich eine etwas geringere Breite auf als die Seitenbügelteile 103, 105 und knickt am Ende ungefähr senkrecht je in eine solche Richtung ab, dass die beiden abknickenden Endstücke der Abschlussteile 105, 106 aufeinander hin gerichtet sind.

Die Seitenbügelteile 104,105 und der in die gleiche Richtung verlaufende Teil der Abschlussteile 105,106 sind zusammen etwa so lang wie die durchschnittliche Tiefe des Kopfes eines Anwenders. Das abknickende Endstück des Abschlussteils vergabelt sich etwa v-förmig in je zwei Teile 105a, 105b und 106a, 106b. Das abgebildete Ausführungsbeispiel hat zum Vorteil, dass das Verengen der Abschlussteile 105, 106 vor der Vergabelung und das v-förmige Vergabeln der Endstücke 105a, 105b und 106a und 106b, den Tragekomfort für den Anwender erhöhen und so eine Möglichkeit erreicht wird, dass das Gestell am Kopf eines Anwenders weitgehend unabhängig von der konkreten Kopfform und Kopfgröße angebracht werden kann. Vorteilhaft ist das Gestell wie in der Abbildung so gefertigt, dass es aus einem Stück besteht und/oder spiegelsymmetrisch zur Mitte des Mittelteils 101 gebaut ist.

Fig. 2 bildet ein Ausführungsbeispiel des Gestells 200 der Vorrichtung ab, wie es auf dem Kopf 299 eines Anwenders angeordnet werden kann. In der abgebildeten Ausführungsform des Gestells umfasst diese einen Mittelteil 201, das etwa so lang wie die durchschnittliche Stirnbreite eines Anwenders ist, und in dessen Mitte ein Abstandshalter 202 angeordnet ist. Dieser Abstandhalter umfasst vorteilhaft wie in der Abbildung gezeigt einen Nasenbügel 202a. Dieser Nasenbügel ist ein sich in zwei Teile verzweigendes Materialstück, dass so positioniert ist, dass er direkt auf dem oberen Nasenrückens des Anwenders 299a angeordnet werden kann und so das Mittelteil 201 über den Abstandhalter 202 und dessen Nasenbügel 202a auf dem Gesicht des Anwender zentriert und fixiert. Die aus Fig. 1 bekannten Seitenbügelteile 103,104 und Abschlussteile 105,106 erhöhen die Stabilität der Befestigung des Gestells am Kopf des Anwenders (nicht ersichtlich aus der Perspektive der Fig. 2). Vorteilhaft kann das Gestell 200 wie in der Abbildung gezeigt, so gefertigt sein, dass es aus einem Stück besteht. Die Anfertigung des Gestells 200 in mehreren aneinander anordenbaren aber getrennten Teilen ist auch ein Ausführungsbeispiel.

Der Abstandshalter der Vorrichtung 202, der fest mit einem Mittelteil 201 der Vorrichtung verbunden ist, ist in einer vorteilhaften Ausführungsform so gestaltet, dass er bei bestimmungsgemäßer Verwendung der Vorrichtung zur Bestrahlung des Auges einen Abstand oder Abstandbereich und/oder einen Winkel oder Winkelbereich der Strahlungsquellen zum Auge fixiert. Die Vorrichtung kann mittels des Abstandhalters zusammen mit einer Sehhilfe, z. B. einer Brille oder einem Monokel, auf einem menschlichen Gesicht angeordnet werden. Dies geschieht in gewisser Ähnlichkeit zur Anbringung einer gewöhnlichen Sehhilfe, unterscheidet sich aber darin, dass der Nasenbügel 202a wie in der Abbildung gezeigt möglichst auf dem oberen Teil des Nasenrückens 299a des Anwenders ruht und einen solchen Winkel über den Abstandhalter 202 mit dem Mittelteil 201 bildet, dass der Anwender zusätzlich eine Sehhilfetragen kann. Dies ist insbesondere dann möglich, wenn die zusätzliche Sehhilfe eine Brille ist, die so gefertigt ist, dass sie auf dem unteren Teil des Nasenrückens aufliegt. Durch das Aufliegen des Nasenbügels 202a der Gestells der Vorrichtung im Gesicht auf dem oberen Teil des Nasenrückens 299a wird erreicht, dass Bestandteile der Vorrichtung, insbesondere das Mittelteil 201, einen definierten Abstand oder Abstandsbereich und/oder Winkel oder Winkelbereich zum Auge oder zu den Augen des Anwenders besitzen. Abstands- und Winkelbereich sind dabei vorteilhaft so gewählt, dass das Sichtfeld des Anwenders trotz Benutzung der erfindungsgemäßen Vorrichtung zusammen mit der Sehhilfe möglichst groß sind. So kann erreicht werden, dass das Sichtfeld bei bestimmungsgemäßer Anwendung der Vorrichtung und bestimmungsgemäßer Anwendung einer Sehhilfe genauso oder nur geringfügig mehr eingeschränkt ist als durch bestimmungsgemäßer Anwendung der Sehhilfe ohne Anwendung der erfindungsgemäßen Vorrichtung. Auf diese Weise kann die erfindungsgemäß Vorrichtung zusammen mit Brille oder Monokel getragen werden, ohne das Sichtfeld des Anwenders stark einzuschränken. Insbesondere kann - unabhängig von der Anwendung einer zusätzlichen Sehhilfe - erreicht werden, dass das Sichtfeld des Anwenders beim bestimmungsgemäßen Anwenden der Vorrichtung nicht wesentlich oder nur im oberen Viertel des Sichtfeldes eingeschränkt ist.

## Patentansprüche

1. Optoelektrische Vorrichtung zur Einstrahlung von Licht in mindestens ein Auge eines Anwenders mit
- mindestens einer Strahlungsquelle, die einen über fünfzig prozentigen Anteil ihrer Strahlungsleistung im Wellenlängenbereich von 400 nm bis 520 nm aufweist,
- einem Gestell (100, 200) zur Befestigung der Strahlungsquelle,
- einer in oder am Gestell (100, 200) angeordneten Stromversorgung, die elektrisch mit der Strahlungsquelle verbunden ist, wobei das Gestell der Vorrichtung einen Mittelteil (101, 202) umfasst, in oder an dem die mindestens eine Strahlungsquelle angeordnet ist, und
- mit einem in der Mitte des Mittelteils (101, 201) mit dem Mittelteil (101, 201) verbundenen Abstandhalter (202), der einen sich in zwei Teile verzweigenden einstückigen Nasenbügel (202a) umfasst, der derart auf dem oberen Teil des Nasenrückens (299a) des Anwenders anordbar ist, dass der Mittelteil (201) über dem Abstandhalter (202) auf dem Nasenrücken (299a) ruht,
- wobei der Anwender zusätzlich zur Vorrichtung eine Brille auf dem Naserücken (299a) tragen kann und der Abstandhalter (202) derart gestaltet ist, dass bei bestimmungsgemäßer Verwendung der Vorrichtung ein Abstand oder Abstandsbereich zwischen der mindestens einen Strahlungsquelle und dem Auge festgelegt ist,
**dadurch gekennzeichnet,**
**dass** die Anordnung der Bestandteile der Vorrichtung dergestalt ist, dass das Sichtfeld des Anwenders bei bestimmungsgemäßem Anwenden der Vorrichtung zusammen mit dem bestimmungsgemäßen Anwenden einer Brille genauso oder nur geringfügig mehr eingeschränkt ist als durch bestimmungsgemäßes Anwenden der Brille ohne die Vorrichtung.

2. Optoelektronische Vorrichtung nach Anspruch 1, **dadurch gekennzeichnet, dass** die Strahlungsquelle eine LED ist.

3. Optoelektronische Vorrichtung nach Anspruch 1 oder Anspruch 2, **dadurch gekennzeichnet, dass** der Mittelteil (101, 201) etwa so breit wie die Stimbreite des Anwenders ist und dass das Gestell (100, 200) an jedem der zwei Enden des Mittelteils (101, 201) jeweils einen etwa senkrecht zum Mittelteil (101, 201) angeordneten Seitenbügel (103, 104) umfasst, wobei die beiden Seitenbügel (103, 104) in die gleiche Richtung weisen, an denen je ein Abschlussteil (106, 105) angeordnet ist, wobei das eine Ende jedes dieser Abschlussteile (106, 105) so von den Seitenbügeln abknickt, dass die Enden der an verschiedenen Seitenbügeln befestigten Abschlussteile (106, 105) aufeinander zuweisen.

4. Optoelektrische Vorrichtung nach Anspruch 3, **dadurch gekennzeichnet, dass** jedes der Abschlussteile (105, 106) in einem direkt an die Seitenbügelteil (103, 104) angrenzenden Bereich eine geringere Breite aufweist als die Seitenbügelteile (103, 104) und sich jedes der Seitenbügelteile v-förmig in je zwei Endstücke (105a, 105b, 106a, 106b) vergabelt.

5. Optoelektrische Vorrichtung nach einem der vorhergehenden Ansprüche, **dadurch gekennzeichnet, dass** mindestens ein Lichtaustrittsfenster für die Strahlung der Strahlungsquelle im oder am Gestell (200, 100) angeordnet ist und die Vorrichtung mindestens eine optische Komponente umfasst, die einen Anteil der Strahlung im zirkadian-wirksamen Emissions-Wellenlängenbereich streut.

6. Optoelektrische Vorrichtung nach einem der vorhergehenden Ansprüche, **dadurch gekennzeichnet, dass** die Vorrichtung einen USB-port umfasst, der eine elektrische Verbindung einer Energiequelle der inneren Stromversorgung zu einer äußeren Stromversorgung bereitstellt, sodass die Energiequelle der inneren Stromversorgung durch die Energie der äußeren Stromversorgung über den USB-port wiederaufladbar ist.

7. Optoelektrische Vorrichtung nach einem der vorhergehenden Ansprüche, **dadurch gekennzeichnet, dass** mindestens eine LED als Signallicht für das Anzeigen eines Betriebszustandes der Vorrichtung im oder am Gestell (200,100) angeordnet ist und das Signallicht ein anderes Strahlungsspektrum als die Strahlungsquelle aufweist.

8. Optoelektrische Vorrichtung nach einem der vorhergehenden Ansprüche, , **dadurch gekennzeichnet, dass** die Energiequelle der Stromversorgung ein Lithium-Akkumulator oder einen Lithium-Polymer-Akkumulator umfasst.

9. Optoelektrische Vorrichtung nach einem der vorhergehenden Ansprüche, **dadurch gekennzeichnet, dass** die Vorrichtung mindestens eine SteuerEinheit umfasst, die die Bestrahlungszeit und/oder Strahlungsintensität der Strahlungsquelle steuert.

10. Optoelektrische Vorrichtung nach einem der vorhergehenden Ansprüche, soweit rückbezogen auf Anspruch 5, **dadurch gekennzeichnet, dass** die Strahlung der mindestens einen Strahlungsquelle durch die optische Komponente so getreut gestreut wird, dass bei Betrieb der Strahlungsquelle weitgehend unabhängig von der Kopfform des Anwenders und/oder von der Kopfhaltung des Anwenders und/oder vom Augenabstand des Anwenders in etwa gleichbleibende Strahldichten und Bestrahlungsstärken auf den Anwender durch die Strahlungsquelle einwirken.

11. Optoelektrische Vorrichtung nach einem der vorhergehenden Ansprüche, **dadurch gekennzeichnet, dass** die Teile, die das Gestell (200, 100) der Vorrichtung ausbilden, in einem Stück miteinander verbunden sind.

## Claims

1. An optoelectric device for radiating light into at least one eye of a user, comprising
- at least one radiation source which has a more than fifty percent fraction of its radiant power in the wavelength range from 400 nm to 520 nm,
- a frame (100, 200) for attachment of the radiation source,
- a power supply arranged in or at the frame (100, 200), which is electrically connected with the radiation source, wherein the frame of the device comprises a middle part (101, 201) in or at which the at least one radiation source is arranged, and
- comprising a spacer (202) connected with the middle part (101, 201) in the center of the middle part (101, 201), which comprises an integral nosepiece (202a) branching into two parts, which can be arranged on the upper part of the nasal bridge (299a) of the user such that the middle part (201) rests on the nasal bridge (299a) above the spacer (202),
- wherein the user can wear glasses on the nasal bridge (299a) in addition to the device and the spacer (202) is designed such that with proper use of the device a distance or distance range between the at least one radiation source and the eye is defined,
**characterized in**
**that** the arrangement of the parts of the device is such that with proper use of the device together with the proper use of glasses the field of view of the user is just as limited as or limited only slightly more than by proper use of the glasses without the device.

2. The optoelectric device according to claim 1, **characterized in that** the radiation source is an LED.

3. The optoelectric device according to claim 1 or claim 2, **characterized in that** the middle part (101, 201) is about as wide as the forehead width of the user and that at each of the two ends of the middle part (101, 201) the frame (100, 200) each comprises a side piece (103, 104) arranged about vertically to the middle part (101, 201), the two side pieces (103, 104) pointing in the same direction, at each of which an end part (106, 105) is arranged, wherein the one end of each of these end parts (106, 105) is bent off from the side pieces such that the ends of the end parts (106, 105) attached to different side pieces point towards each other.

4. The optoelectric device according to claim 3, **characterized in that** in a region directly adjoining the side piece parts (103, 104) each of the end parts (105, 106) has a smaller width than the side piece parts (103, 104) and each of the side piece parts bifurcates into two end pieces (105a, 105b, 106a, 106b) each in a v-shaped manner.

5. The optoelectric device according to any of the preceding claims, **characterized in that** at least one light exit window for the radiation of the radiation source is arranged in or at the frame (200, 100) and the device comprises at least one optical component which scatters a part of the radiation in the circadian-effective emission wavelength range.

6. The optoelectric device according to any of the preceding claims, **characterized in that** the device comprises a USB port which provides an electrical connection of an energy source of the inner power supply to an outer power supply, so that the energy source of the inner power supply is rechargeable by the energy of the outer power supply via the USB port.

7. The optoelectric device according to any of the preceding claims, **characterized in that** at least one LED is arranged in or at the frame (200, 100) as signal light for indicating an operating condition of the device and the signal light has a radiation spectrum other than the radiation source.

8. The optoelectric device according to any of the preceding claims, **characterized in that** the energy source of the power supply comprises a lithium accumulator or a lithium-polymer accumulator.

9. The optoelectric device according to any of the preceding claims, **characterized in that** the device comprises at least one control unit which controls the irradiation time and/or radiation intensity of the radiation source.

10. The optoelectric device according to any of the preceding claims, as far as related to claim 5, **characterized in that** the radiation of the at least one radiation source is scattered by the optical component such that in operation of the radiation source approximately constant beam densities and irradiation intensities act on the user through the radiation source independent of the head shape of the user and/or of the head position of the user and/or of the eye distance of the user.

11. The optoelectric device according to any of the preceding claims, **characterized in that** the parts which form the frame (200, 100) of the device are connected with each other in one piece.

## Revendications

1. Dispositif optoélectrique pour irradiation d'une lumière dans au moins un oeil d'un utilisateur avec
- au moins une source de rayonnement qui présente une proportion supérieure à cinquante pour cent de sa capacité de rayonnement dans une plage de longueurs d'ondes de 400 nm à 520 nm,
- un châssis (100, 200) pour la fixation de la source de rayonnement,
- une alimentation de courant disposée dans ou sur le châssis (100, 200) qui est reliée électriquement à la source de rayonnement, le châssis du dispositif comprenant une partie médiane (101, 202) dans ou sur laquelle est disposée au moins une source de rayonnement, et
- un élément d'espacement (202) relié à la partie médiane (101, 201) au milieu de la partie médiane (101, 201), qui comprend une barrette nasale (202a) d'un seul tenant se ramifiant en deux parties, qui peut être placé sur la partie supérieure de l'arête du nez (299a) de l'utilisateur de telle sorte que la partie médiane (201) repose sur l'élément d'espacement (202) sur l'arête du nez (299a),
- l'utilisateur pouvant porter, en plus du dispositif, des lunettes sur l'arête du nez (299a) et l'élément d'espacement (202) étant conçu de telle sorte que lors de l'utilisation conventionnelle du dispositif, un intervalle ou une zone d'intervalle entre au moins la source de rayonnement et l'oeil soit déterminé(e),
**caractérisé en ce que**
la disposition des composants du dispositif est conçue de telle sorte que le champ de vision de l'utilisateur lors d'une utilisation conventionnelle du dispositif conjointement avec l'utilisation conventionnelle de lunettes est identique ou uniquement provisoirement plus limitée que lors de l'utilisation conventionnelle de lunettes, sans le dispositif.

2. Dispositif optoélectrique selon la revendication 1, **caractérisé en ce que** la source de rayonnement est une DEL.

3. Dispositif optoélectrique selon la revendication 1 ou la revendication 2, **caractérisé en ce que** la partie médiane (101, 201) est à peu près aussi large que la largeur du front de l'utilisateur et **en ce que** le châssis (100, 200) sur chacune des deux extrémités de la partie médiane (101, 201) comprend respectivement une barrette latérale (103, 104) disposée à peu près perpendiculairement à la partie médiane (101, 201), les deux barrettes latérales (103, 104) présentant la même direction, sur lesquelles respectivement, une partie terminale (106, 105) est disposée, une extrémité de chacune de ces parties terminales (106, 105) bifurquant des arêtes latérales de telle sorte que les extrémités des parties terminales (106, 105) fixées à différentes arêtes latérales se tournent l'une vers l'autre.

4. Dispositif optoélectrique selon la revendication 3, **caractérisé en ce que** chacune des parties terminales (105, 106) présente dans une zone directement adjacente à la partie de barrette latérale (103, 104), une largeur plus réduite que les parties de barrette latérale (103, 104) et chacune des parties de barrette latérale se ramifie en forme de V en deux éléments terminaux respectifs (105a, 105b, 106a, 106b).

5. Dispositif optoélectrique selon l'une des revendications précédentes, **caractérisé en ce qu'**au moins une fenêtre de sortie de lumière est disposée pour l'irradiation de la source de rayonnement dans ou sur le châssis (200, 100) et le dispositif comprend au moins un composant optique qui diffuse une partie du rayonnement dans une plage de longueurs d'ondes d'émission efficaces sur le plan circadien.

6. Dispositif optoélectrique selon l'une des revendications précédentes, **caractérisé en ce que** le dispositif comprend un port USB qui met à disposition une liaison électrique d'une source d'énergie de l'alimentation de courant interne à une alimentation de courant externe de sorte que la source d'énergie de l'alimentation de courant interne puisse être rechargeable par l'énergie de l'alimentation de courant externe par le port USB.

7. Dispositif optoélectrique selon l'une des revendications précédentes, **caractérisé en ce qu'**au moins une DEL est disposée en tant que lumière de signal pour indiquer un état de fonctionnement du dispositif dans ou sur le châssis (200, 100) et la lumière de signal présente un autre spectre de rayonnement que la source de rayonnement.

8. Dispositif optoélectrique selon l'une des revendications précédentes, **caractérisé en ce que** la source d'énergie de l'alimentation de courant comprend un accumulateur lithium ou un accumulateur lithium - polymère.

9. Dispositif optoélectrique selon l'une des revendications précédentes, **caractérisé en ce que** le dispositif comprend au moins une unité de commande qui commande le temps d'irradiation et/ou l'intensité de rayonnement de la source de rayonnement.

10. Dispositif optoélectrique selon l'une des revendications précédentes, selon la revendication 5, **caractérisé en ce que** le rayonnement d'au moins une source de rayonnement est dispersée par les composants optiques de façon si diffuse que lors du fonctionnement de la source de rayonnement, ils influent largement indépendamment de la forme de la tête de l'utilisateur et/ou du maintien de la tête de l'utilisateur et/ou de la distance oculaire de l'utilisateur, à peu près dans des densités de rayon et des puissances de rayonnement constantes sur l'utilisateur par la source de rayonnement.

11. Dispositif optoélectrique selon l'une des revendications précédentes, **caractérisé en ce que** les parties qui constituent le châssis (200, 100) du dispositif sont reliées les unes aux autres en une pièce.
